# EUROPEAN PATENT SPECIFICATION

(11) **EP 3 436 033 B1**
(45) Date of publication and mention of the grant of the patent: **12.02.2020**
(21) Application number: 17717972.8
(22) Date of filing: 29.03.2017
(51) Int. Cl.: A61K 33/06, A61P 7/08

(54) **A POLY-OXYGENATED METAL HYDROXIDE COMPRISING A CLATHRATE THAT INCREASES OXYGEN LEVELS IN MAMMALIAN TISSUES**
POLYOXYGENIERTES METALLHYDROXID ENTHALTEND EIN KLATHRAT, DAS DIE SAUERSTOFFVERSORGUNG VON SÄUGETIERGEWEBEN ERHÖHT
HYDROXIDE POLYOXYGÉNÉ COMPRENANT UN CLATHRATE QUI AUGMENTE LE NIVEAU D'OXYGÈNE DANS LES TISSUS MAMMAIRES

(30) Priority: 30.03.2016 US 201662315524 P; 21.06.2016 US 201615188586
(43) Date of publication of application: 06.02.2019
(73) Proprietor: Baylor University, Waco, TX 76798-7014 (US)
(72) Inventor: BRUCE, Erica, D., Hewitt, TX 76643 (US); SAYES, Christie, McGregor, TX 76657 (US); WOODMANSEE, John,W., Frisco, TX 75034 (US)
(74) Representative: Hartley, Lucinda Jane
(86) International application number: PCT/US2017/024717
(87) International publication number: WO 2017/172893

(56) References cited:
- WO-A2-2009/082449
- ES-A1- 8 506 182
- Peijin Yang: "Investigating the efficacy of a novel therapeutic to mitigate traumatic brain injury: contributions of environmental exposures to overall healing", Internet , 30 July 2015 (2015-07-30), XP002770281, Retrieved from the Internet: URL:https://baylor-ir-tdl.org/baylor-ir/ha ndle/2104/9466 [retrieved on 2017-05-16]
- Anonymous: "News from and of our Classmates, Continued", Internet , October 2013 (2013-10), XP002770282, Retrieved from the Internet: URL:https://alumni.culver.org/document.doc ?id=517&chid=63 [retrieved on 2017-05-16]

## Description

### TECHNICAL FIELD

The disclosure relates generally to a composition, and a method of generating a poly-oxygenated metal hydroxide material comprising a clathrate containing oxygen gas (O_{2(g)}) molecules, and creating homogenous particles suitable for advanced therapies when delivered to mammals, including humans and animals. Principal delivery methods include topical, oral, anal, vaginal, inhalation, intramuscular injection, and intravenous delivery.

### BACKGROUND

Oxygen is one of the fundamental building blocks of life. Oxygen sustains life, but it also has therapeutic (i.e. healing) powers when delivered topically to tissue, orally for digestion, anally, vaginally, aerosolized for inhalation, injected to intramuscular tissue, intravenously to the blood circulatory system, and other delivery methods. Conventional oxygen therapies are commonly comprised of a gaseous delivery of oxygen (i.e. O₂) in chambers, such as hyperbaric oxygen therapy (HBOT). However, the concentration of oxygen delivered by gas is rather small, and the chambers are both expensive and not widely available.

A poly-oxygenated aluminum hydroxide, such as manufactured by Hemotek, LLC of Plano, Texas as Ox66™, is a revolutionary product that has been proven to have therapeutic benefits. Ox66™ is provided in powder form and is described as a non-homogenous size particle population, typically ranging from about 50 to 800 micrometers (µm).

### SUMMARY

A composition of a poly-oxygenated metal hydroxide comprising a clathrate containing oxygen gas molecules having particles sized of less than or equal to 3 µm in diameter, such that they can deposit into the deep airway ducts and diffuse evenly within the alveolar or gas exchange regions of the lung to treat internal burns.

One exemplary embodiment is delivering poly-oxygenated metal hydroxide particles intravenously as a resuscitative fluid, and to treat diseases of organs when the diameter of the particles is in the range of 250 nm to 1000 nm. Particles having critical diameters between 250 to 1000 nm will stay in the capillary, vein, or artery linings of the circulatory system and not passively diffuse past the lining into surrounding tissue.

### BRIEF DESCRIPTION OF THE FIGURES

Figure 1 is a scanning electron microscopy (SEM) image of a single 50 micrometer (µm) Ox66™ particle;
Figure 2 is a graphic art image of the jagged shaped Ox66™ particle population;
Figure 3 depicts one exemplary process used to create nano-engineered Ox66™ nanoparticles to exploit the physical and chemical properties of each particle-type;
Figure 4 illustrates three different graphs modeling the effect of Ox66™ particle size when varying (A) rotation rate, (B) grinding ball size, and (C) rotation time; and
Figures 5-7 are scanning electron microscopy (SEM) images showing the nano-engineered Ox66™ particles at different image magnifications having particle diameters at 3 µm and below.

### DETAILED DESCRIPTION

The following description of example embodiments provides information that enables a person skilled in the art to make and use the subject matter set forth in the appended claims, but may omit certain details already well-known in the art. The following detailed description is, therefore, to be taken as illustrative and not limiting. Objectives, advantages, and a preferred mode of making and using the claimed subject matter may be understood best by reference to the accompanying drawings in conjunction with the following detailed description of illustrative embodiments.

The example embodiments may also be described herein with reference to spatial relationships between various elements or to the spatial orientation of various elements depicted in the attached drawings. In general, such relationships or orientation assume a frame of reference consistent with or relative to a patient in a position to receive treatment. However, as should be recognized by those skilled in the art, this frame of reference is merely a descriptive expedient rather than a strict prescription.

Engineered nanoparticles are routinely defined as particles with sizes between about 1 and 1000 nm that show physical or chemical properties that are not found in bulk samples of the same material.

Dissolved oxygen refers to micrometer or nanometer sized bubbles of gaseous oxygen (mixed in water or other aqueous solution) made bioavailable to organisms, animals, or humans for respiration.

Aqueous medium means pertaining to, related to, and similar to water (the most common solvent on Earth).

The inventive concepts disclosed and claimed herein relate generally to a nanometer-sized composition and process for forming poly-oxygenated metal hydroxide particles having a diameter of less than or equal to 3 µm. These engineered hydroxide particles having a diameter of less than or equal to 3 µm enable numerous revolutionary applications and treatments that provide significant achievements in bioscience. Through research and clinical studies, these particles have been proven to treat body conditions of mammals, including humans and animals, with astounding success and efficiency. These engineered hydroxide particles having a diameter of less than or equal to 70 nm do not create an immune response of the mammal.

Ox66™ particles are non-toxic poly-oxygenated aluminum hydroxide complexes stored in either a 1-99% by weight aqueous solution or as a dried powder, and are available from Hemotek LLC of Piano, TX. The material is non-flammable, water-soluble, and slightly basic. The Ox66™ composition provided from Hemotek, LLC is in powder form and is described as a non-homogenous size particle population, typically ranging from about 50 to 800 micrometers (µm). These particles can also be described as a non-corrosive and non-vapor producing powder. Its appearance is white to slightly blue as a powder with mass but very little weight (i.e. one gallon weighs less than 4.3 ounces) or a clear slightly viscous liquid when placed in an aqueous suspension.

Ox66™ is an oxygen gas carrying powder that contains about 66% oxygen, and is a true clathrate that is a lattice-like structure that provides large areas capable of capturing and holding oxygen gas molecules.

Ox66™ exists under STP (standard temperature and pressure) as a clathrate. A clathrate is a chemical substance consisting of a lattice that traps or contains molecules. The molecules trapped or contained within the Ox66™ clathrate are oxygen gas (O_{2(g)}). The molecular formula of Ox66™ is Al₁₂H₄₂O₃₆, which mathematically reduced is Al(OH)₃•6O₂. The 6 free oxygen gas molecules (O_{2(g)}) are separate from the oxygen molecules covalently bound in the hydroxide complex.

According to exemplary embodiments of this disclosure, through research, studies and clinical studies, it has been discovered that engineering the Ox66™ particles to have diameter sizes at or below 3 µm opens up significant and revolutionary new opportunities for oxygen therapy. Providing particles having diameters of 3 µm or less is critical to achieve numerous new applications, such as by oral, nasal, intravenous, anal, vaginal, and topical delivery, to treat conditions and diseases in revolutionary ways. Particles having a diameter size from 1 to 3000 nm deposit into the deep airway ducts and diffuse evenly within the alveolar or gas exchange regions of the lung.

Another exemplary embodiment is delivery, by aerosol when inhaled, for absorption of the poly-oxygenated metal hydroxide particles through the lung tissue when the particles are reduced to 250 nm and less. Such an application effectively treats internal burns.

One exemplary embodiment is delivering poly-oxygenated metal hydroxide particles intravenously as a resuscitative fluid, and to treat diseases of organs when the diameter of the particles is in the range of 250 nm to 1000 nm. Particles having critical diameters between 250 to 1000 nm will stay in the capillary, vein, or artery linings of the circulatory system and not passively diffuse past the lining into surrounding tissue.

A remarkable example is delivering the poly-oxygenated aluminum hydroxide particles intravenously to treat traumatic brain injury (TBI) when the diameter of the particles is reduced to about 10nm and less so that the particles can traverse the brood brain barrier (BBB). This application can also be used to treat strokes, chronic traumatic encephelopathy (CTE), and perhaps even cancer. Ongoing research at Baylor University, the applicant of this application, continues to discover and prove numerous revolutionary uses for nano-sized poly-oxygenated aluminum hydroxide.

There is a significant biophysical difference between a 50 µm particle and a 3 µm particle. After intravenous administration, 50 µm particles are larger and have more mass than 3 µm particles, therefore they tend to absorb onto the linings of the veins. Three (3) µm particles stay in circulation much longer, have much less mass, and have higher surface area. After inhalation administration, 50 µm diameter particles deposit in the oral or nasal cavity and do not reach even the upper airways of the lung. Three (3) µm diameter particles are small enough to deposit in the very deep lung and perfuse out to the lung lining, and thus particle sizing is critical. After topical administration, 50 µm diameter particles tend to stay on the surface of the epidermis and eventually wash off the skin completely. Three (3) µm diameter particles penetrate through the epidermis and dermis layers of the skin and reside in the subcutaneous layer of the skin. After oral administration, 3 µm diameter particles absorb through the lining of the esophagus and stomach. Fifty (50) µm diameter particles reside in the stomach for up to 4 hours, dissolve (or break-down) and lose their oxygen carrying capability.

Another exemplary embodiment of this disclosure includes increasing the oxygen content of fluids with nanometer-sized Ox66™ particles, such as water, sports drinks, and nutritional drinks, which provides many benefits and applications. The nanometer-sized Ox66™ particles have been clinically shown to pass through the stomach, duodenum, and intestinal walls into the bloodstream of the body, and are not simply absorbed by the stomach lining. One method for increasing the dissolved oxygen content in an aqueous medium includes sparging the aqueous medium with air, oxygen or oxygen-enriched air.

In another exemplary embodiment of this disclosure, the nanometer-sized Ox66™ particles, either as a powder or in a carrier such as a gel or lotion, have also been clinically proven to increase the level of localized oxygen in injured tissues to accelerate the healing process.

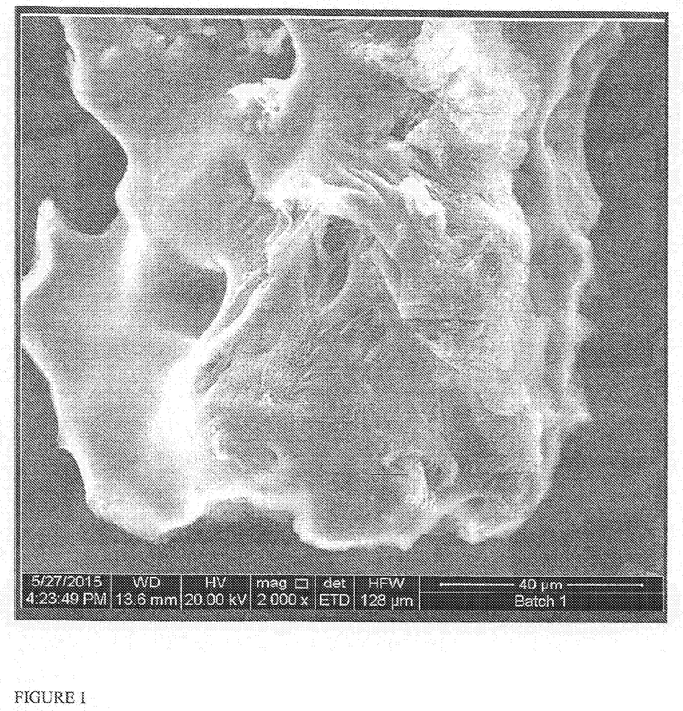

Figure 1 is a scanning electron microscopy (SEM) image of a single 50 micrometer (µm) Ox66™ particle. A 50 µm particle is easily aerosolized, but it is well outside the critical respirable range of 1-3 µm. The 50 µm particle has little density due to its chemical composition and its porosity.

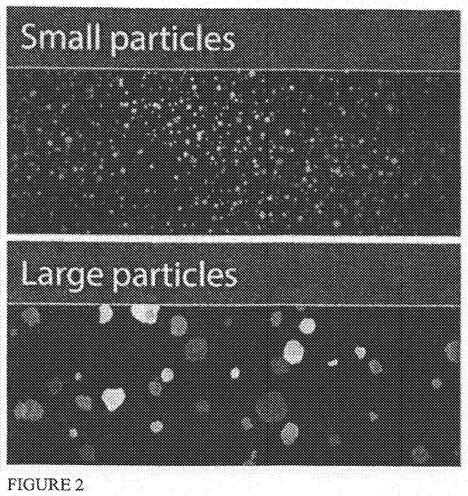

Figure 2 is a graphic art image of the jagged shaped Ox66™ particle population.

Controlled milling is defined as a machining procedure using vessels accelerating in a rotary or planetary motion to decrease the size of the primary particles from micrometer sized to nanometer sized materials. Milling covers a wide array of procedures, operations, tools, and machines. The resultant nanometer sized particle can be accomplished using small instruments or large milling machines. Example milling instruments include: "milling machine", "machining centers", or "multitasking machines".

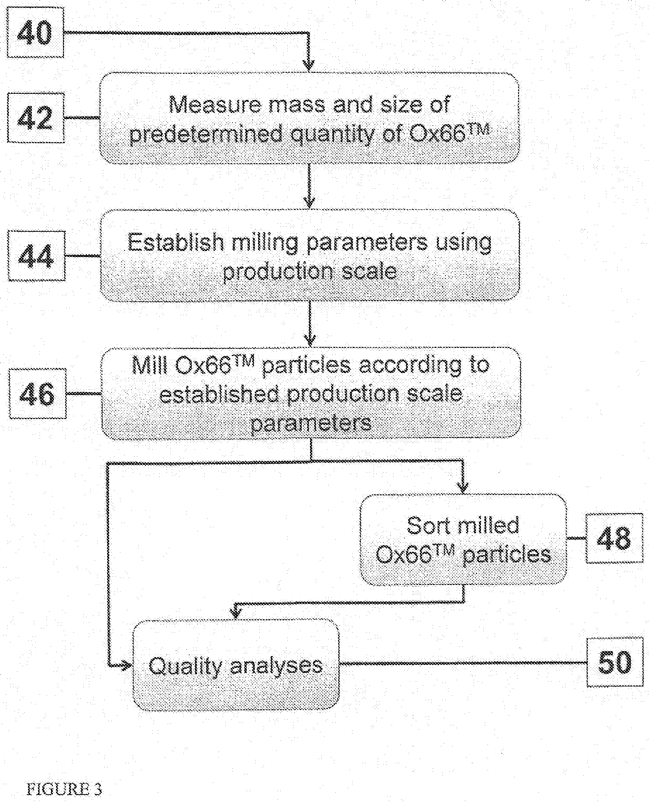

Referring to Figure 3, there is shown an exemplary process at 40 for forming nanosized Ox66™ particles having diameter sizes of 3 µm or less using a planetary motion milling machine.

At step 42, a predetermined quantity of the quality assured Ox66™ powdered material is measured, and placed in a container.

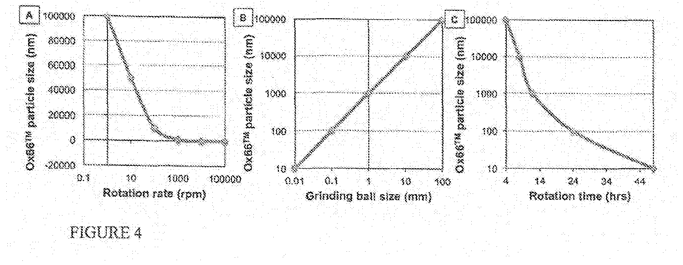

At step 44, a milling scale is used to establish parameters of the generating particles having a diameter of 3 µm or less, such as shown in Figure 4, both for small-scale production as well as mass production. The milling procedure is dependent upon the features of the ball mill, which may be a planetary motion device, such as Retsch Planetary Ball Mill PM 100, 200, or 400 or United Nuclear Scientific Equipment 'Hobby" Ball Mill. The milling procedure identifies several variables, including a quantity of Ox66™ material, the rotation rate, the size of the milling beads, the type of milling beads, and the time of milling to achieve desired size of the Ox66™ particles. For example, the rotation rate may be for at least 1 minute up to 1,440 minutes at a rotation rate of at least 1 00 up to 10,000 rotations per minute.

Figure 4 includes three different graphs modeling the effect of Ox66™ particle size when varying (A) rotation rate, (B) grinding ball size, and (C) rotation time. As rotation rate, measured in rotations per minute (rpm) increases, particle size decreases. As grinding ball size, measured in millimeters (mm) decreases, particle size decreases. As rotation time, measured in hours (hrs) increases, particle size decreases.

At step 46, the predetermined quantity of Ox66™ particles are then milled in a controlled manner in a planetary motion ball mill, according to the milling procedure to achieve a desired size of the Ox66™ particles. The Ox66™ particles are milled or ground down under high energy in the presence of a milling media, such as highly reticulated polystyrene or zirconium milling beads. The Ox66™ particles are recirculated, re-milling them until a consistent product is generated.

Optionally, at step 48, additional sorting may be performed to create homogenous size particles, such as using sieves as will be described shortly.

At step 50, the milled Ox66™ particles are subjected to quality analysis to confirm sizing and consistency. If the Ox66™ particles are not consistent, they may be further milled to achieve the desired sizing.

The milling media can also abrade under the conditions of milling, so care is taken such that significant contamination of the nanosuspension by the milling media does not occur. Nanosuspension is defined as a submicron colloidal dispersion of drug particles.

The resultant Ox66™ particles have a primary critical particle size of 3 µm or less. In one exemplary embodiment, the reduced size particles are then separated into homogeneous sizes in an effort to exploit the physical and chemical properties of each particle-type. Sieves can be used to sort out particles by diameter sizes to create homogenous sizes of particles, such as using sieve shakers manufactured by Endecotts Ltd of London, UK. Different size sieve filters are used to obtain selected particle sizes.

One size of particles is particularly beneficial for treating a particular body condition, such as 10 nm diameter particles to treat traumatic brain injury (TBI). Another homogenous size of particles may be beneficial for providing a resuscitative fluid (RF) to increase the tissue oxygenation (PO₂), such as using 35 to 70 nm diameter particles which do not trigger an immune response. Generating nanometer sized particles increases the *in vivo* (i.e. in a whole, alive organism) dissolution rate and fraction absorbed to increases oral bioavailability.

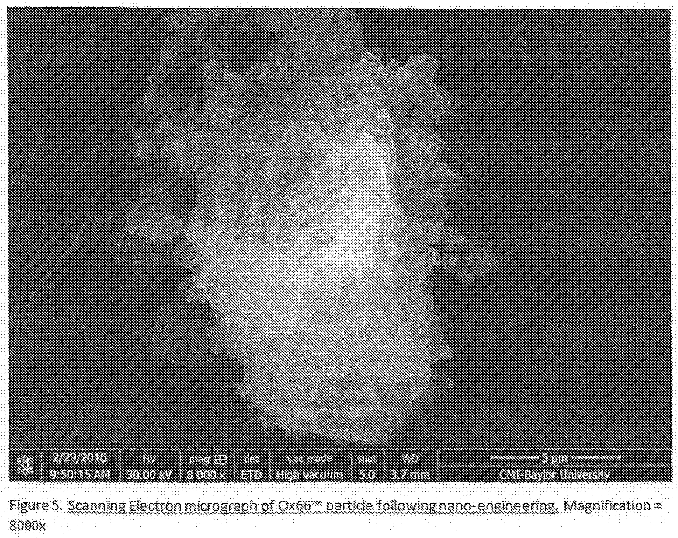

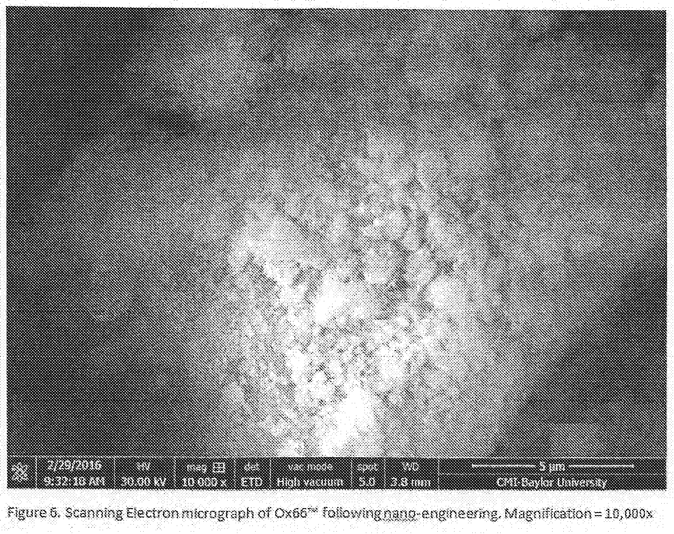

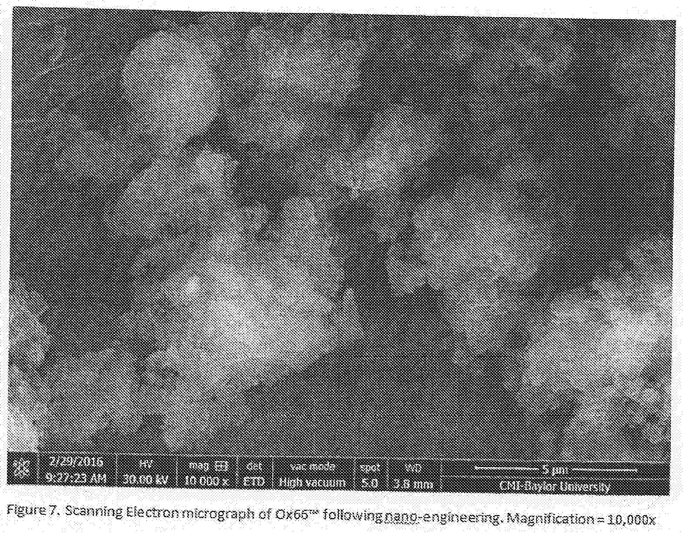

Figures 5-7 are scanning electron microscopy (SEM) images showing the nano-engineered Ox66™ particles at different image magnifications, showing the particle diameters at 3 µm and below.

The pharmaceutical preparation of nanomaterial-based dosage forms is encouraged by a number of pharmaceutical drivers; for compounds whose water solubility or dissolution rate limits their oral bioavailability, size reduction into the nanometer size domain can increase in vivo dissolution rate and fraction absorbed.

The process to generate a homogeneous nanometer size particle population can also be of use in the design of parenteral dosage forms wherein poorly soluble drugs can be "milled" to a specified size and size range resulting in not only useful bioavailability but also sustained release features.

The development of drug particles within the nanometer size regime of 1 to 1000 nm involves a top-down approach in which the active ingredient is milled (or otherwise subjected to particle reduction strategies) in either an aqueous environment or in a dry formulation; top-down strategies are considered more controllable and more robust as a function of process and design space for this type of manipulation.

The appended claims set forth novel and inventive aspects of the subject matter described above, but the claims may also encompass additional subject matter not specifically recited in detail. For example, certain features, elements, or aspects may be omitted from the claims if not necessary to distinguish the novel and inventive features from what is already known to a person having ordinary skill in the art. Features, elements, and aspects described herein may also be combined or replaced by alternative features serving the same, equivalent, or similar purpose without departing from the scope of the invention defined by the appended claims.

## Claims

1. A composition, comprising:
a poly-oxygenated metal hydroxide having particle sizes of less than or equal to 3 µm in diameter, wherein the poly-oxygenated metal hydroxide comprises a clathrate containing free oxygen gas (O₂) molecules.

2. The composition as specified in Claim 1, wherein the poly-oxygenated metal hydroxide has particle sizes of less than or equal to 1 µm in diameter.

3. The composition as specified in Claim 1 wherein the poly-oxygenated metal hydroxide comprises a poly-oxygenated aluminum hydroxide.

4. The composition as specified in Claim 1, wherein the poly-oxygenated metal hydroxide is homogeneous.

5. The composition as specified in Claim 1 wherein the poly-oxygenated metal hydroxide is non-toxic to a mammal.

6. The composition as specified in Claim 5 for use in medicine.

7. The composition as specified in Claim 6 for use in a method of intravenous administration to a mammal.

## Patentansprüche

1. Zusammensetzung, umfassend:
ein polyoxygeniertes Metallhydroxid, das Teilchengrößen mit einem Durchmesser kleiner gleich 3 µm aufweist, wobei das polyoxygenierte Metallhydroxid ein Clathrat umfasst, das freie Sauerstoffgasmoleküle (O₂-Moleküle) enthält.

2. Zusammensetzung nach Anspruch 1, wobei das polyoxygenierte Metallhydroxid Teilchengrößen mit einem Durchmesser kleiner gleich 1 µm aufweist.

3. Zusammensetzung nach Anspruch 1, wobei das polyoxygenierte Metallhydroxid ein polyoxygeniertes Aluminiumhydroxid umfasst.

4. Zusammensetzung nach Anspruch 1, wobei das polyoxygenierte Metallhydroxid homogen ist.

5. Zusammensetzung nach Anspruch 1, wobei das polyoxygenierte Metallhydroxid für ein Säugetier nicht toxisch ist.

6. Zusammensetzung nach Anspruch 5 zur Verwendung in der Medizin.

7. Zusammensetzung nach Anspruch 6 zur Verwendung in einem Verfahren zur intravenösen Verabreichung an ein Säugetier.

## Revendications

1. Composition comprenant:
un hydroxyde métallique poly-oxygéné ayant des particules d'une taille inférieure ou égale à 3 µm en diamètre, où l'hydroxyde métallique poly-oxygéné comprend un clathrate contenant des molécule d'oxygène gazeux (O₂) libres.

2. Composition telle que spécifiée à la revendication 1, où l'hydroxyde métallique poly-oxygéné a des particules d'une taille inférieure ou égale à 1 µm en diamètre.

3. Composition telle que spécifiée à la revendication 1, où l'hydroxyde métallique poly-oxygéné comprend un hydroxyde d'aluminium poly-oxygéné.

4. Composition telle que spécifiée à la revendication 1, où l'hydroxyde métallique poly-oxygéné est homogène.

5. Composition telle que spécifiée à la revendication 1, où l'hydroxyde métallique poly-oxygéné n'est pas toxique pour un mammifère.

6. Composition telle que spécifiée à la revendication 5 pour une utilisation en médecine.

7. Composition telle que spécifiée à la revendication 6 pour une utilisation dans une méthode d'administration intraveineuse à un mammifère.
